# EUROPEAN PATENT SPECIFICATION

(11) **EP 1 433 442 B1**
(45) Date of publication and mention of the grant of the patent: **14.11.2007**
(21) Application number: 02080489.4
(22) Date of filing: 24.12.2002
(51) Int. Cl.: A61F 2/14, G02C 7/04

(54) **Cosmetic eye implant**
Kosmetisches Augenimplantat
Implant oculaire cosmétique

(43) Date of publication of application: 30.06.2004
(73) Proprietor: Medical Technology Transfer Holding B.V., 3071 MJ Rotterdam (NL)
(72) Inventor: Melles, Gerrit Reinold Jacob, 3071 MJ Rotterdam (NL)
(74) Representative: Prins, Adrianus Willem

(56) References cited:
- WO-A-97/48004
- DE-A- 19 850 807
- US-A- 5 617 154
- US-A- 5 713 957
- US-B1- 6 280 469

## Description

The invention relates to an eye implant. The invention more specifically relates to a cosmetic eye implant, especially one suitable for implantation in the eye.

The invention further relates to the use of a bio compatible element as an eye implant, more specifically a cosmetic eye implant.

It is known to use implants in vertebrate such as a human body for both therapeutical and cosmetic purposes. The latter sort are commonly known as piercings. Piercings are normally constituted of for example stainless steel elements such as earrings and are easily releasably connected to the human body by insertion through an artificial opening in the skin, for example an earlobe or bellybutton. Part of the element stays outside the skin and is therefore visible, providing for the desired cosmetic effect.

DE 198 508 07 discloses an artificial iris system for implantation in an eye of a vertebrate, for restoring the natural appearance of an eye and imitating the natural eye function of such eye, including the normal light dependent shutter function of the pupil. Claim 1 is delimited over DE19850807.

US 5 713 957 discloses corneal onlays for use in surgical implantation in or on the cornea of a mammal having an optical axis region with optical characteristics which provide visual acuity therethrough, which have a porosity sufficient to allow passage therethrough of tissue fluid. These onlays are adapted for epithelial recolonization and are intended for correcting the optical property of an eye.

The inventor of the present invention has had the surprising insight that implants can also be used for inter alia cosmetic purposes in or on an eye of a vertebrate. To this end an eye implant is provided according to the invention, which is characterised by the features of claim 1.

By providing a visible implant in or on the eye, especially visible in a frontal view of the eye, on or through the conjunctiva and/or the cornea, an implant is provided which will be highly visible to any body looking at or into said eye. Any encounter with a person or animal having such implant would lead almost automatically to a view of said implant. Therefore an implant according to the present invention will have a high cosmetic impact. Visible has to be understood as meaning that the implant will be noticeable from a relatively large distance, when the relevant eye is opened.

Biocompatible has to be understood as compatible for ophthalmic use, especially for use in or on an eye, without resulting in inducement of intra ocular reaction such as siderosis bulbi or medical complications in the broader sense. Opaque has to be understood as not fully transparent and/or reflective for light within the visual spectrum. Reflective has to be understood as reflecting at least part of the visual spectrum. Flat has to be understood as having a thickness in a first direction which is considerably less than the measurements perpendicular to said first direction. Cosmetic has to be understood as having substantially no medical purpose, such as a chemical or medicinal function or a vision aiding function.

It is known to use artificial lenses in the eye, for replacement of the crystalline lense which is effected by cataract surgery. Such implants are however necessarily highly transparent. It is furthermore known to use contact lenses which are temporarily positioned on the cornea, or phakic intraocular lenses that are positioned inside the eye. These also are necessarily highly transparent and must cover at least the pupil and at least part of the cornea. These known implants are therefore relatively large and not visible during use.

An eye implant according to the present invention is dimensioned such, in size, form, weight and position during use that the normal eye functions are not impaired by said implant. Preferably the thickness of the element is such that during use no significant tissue deformation occurs, whereas ocular movement is not significantly hindered and the visual function of the eye is not impaired. The thickness is for example less than approximately 0.5 mm, more specifically less than 0.3 mm and preferably between 1 and 250 micron. The outer dimensions, perpendicular to said thickness are less than 5 mm and preferably between 0.5 and 4 mm.

In a further preferred embodiment an eye implant element according to the present invention is designed to be introduced into the eye, to which end the element is provided with an outer edge or periphery which is substantially rounded or otherwise smoothed and made non-cutting. Such a substantially rounded outer periphery has the advantage that after implantation the danger of for example inadvertent damage to or inflammation of the adjacent ocular tissue is prevented. Introduction of the implant into the eye has the advantage that during use no contact exists between the element and for example the eye lids, preventing the risk of impairment of the normal eye movements and other behaviour, whereas irritation of the tissue of for example the eye lids and the outer side of the conjunctiva and/or cornea is prevented. Moreover, excessive tear forming is also prevented. An other advantage is that the element is fully covered, preventing that it can for example get hooked behind artefacts of be pulled out inadvertently.

An element for an eye implant according to the present invention is preferably mainly made of one or more materials chosen from the group of platinum, platinum-iridium, titanium, nitinol, gold, silver, glass, polymethylmethacrylate, stone, jewellery stone, sand, silica, porcelain, hair, cilia, and (colored) visco elastic materials, for example hyaluronic acid. These have all been proven to be bio compatible as defined here above. Obviously also other materials, combinations of materials analoys, mixtures thereof can be used if biocompatible, that is suitable for ophthalmic use, especially intra ocular. The material or combination of materials is preferably strerilisable, such that it can either be sterilised directly prior to implantation or earlier, in which case it can be packed sterile in a container such as a box or pouch.

The present invention further relates to the use of an element as here before described as an eye implant, especially to be implanted on or in the conjunctiva, between the conjunctiva and the sclera, in the sclera or in or under the cornea.

In an element according to the present invention active elements may be used such as electronic of magnetic transponders. This may serve for example identification or locating purposes.

The present invention further discloses the use of the implant for an eye of a vertebrate, especially on or in the conjunctiva, between the conjunctiva and the sclera, in the sclera, between the sclera and the cornea or in or under the cornea. Surprisingly it has been found that such element can easily be placed and will be directly visible to any body looking into said eye, for example to check the presence of the transponder. Moreover such element will not significantly impair the normal functions of the eye. Said element is preferably an element as previously disclosed. Obviously said implant can be positioned in various areas, especially in areas as disclosed in claims 19 - 27.

By using a method according to the present invention cosmetic improvements to the eye can be obtained in a relatively easy manner. Surprisingly it has been found that an element as previously disclosed can be inserted in an eye, at various levels and covered by eye tissue, without impairing the normal eye functions and without irritation to the outer layer of the conjunctiva and/or the eye lid. Especially placing such element in the conjunctiva or between the conjunctiva and the sclera could be performed by a non-medical person such as a tatooist or an optician.

In the sub claims further advantageous embodiments of products according to the present invention are given.

For a better understanding of the present invention embodiments of an eye implant, use thereof, methods for implanting an element in an eye and eyes obtained therewith will be described hereafter, in relation to the accompanying drawings, in which shows:
Figure 1A - C three embodiments of an eye according to the present invention, provided with an eye implant, visible in frontal view;
Figure 2 in cross section schematically a human eye;
Figure 3 in enlarged cross section part of the eye as shown in figure 2;
Figure 4, schematically in frontal view a number of various shapes of eye implants according to the present invention, in enlarged views;
Figure 5 in even further enlarged view a cross section along the line V-V in figure 4;
Figure 6A - D schematically four steps of a method according to the present invention for placing an eye implant; and
Figure 7A - D four different possible positions of an eye implant according to the present invention.

In this description identical or corresponding parts have identical or corresponding reference signs. In the description and drawings embodiments are given as mere examples and should in no way be understood as limiting the scope of the present invention.

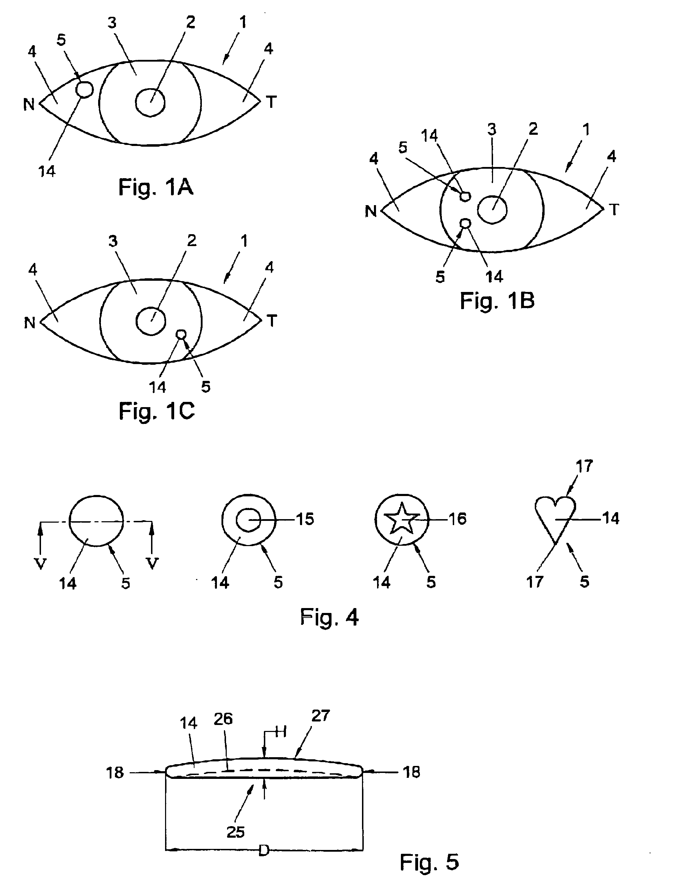

In figure 1A - C an eye 1 is shown, schematically, in frontal view. The eye 1 shown is a human eye but could also be the eye of another vertebrate, such as a domestic or farm animal. The eye comprises a pupil 2 with surrounding iris 3 and eye white 4 on both the temporal and nasal sides. The eye can either be a left or a right eye. In figure 1 the temporal side T is shown at the right side, the nasal side N at the left side.

In figure 1A an eye implant 5 is shown at the nasal side and spaced apart from the iris 3. In figure 1B two eye implants 5 are shown, in frontal view in front of the iris 3, next to the pupil 2, whereas in figure 1C an eye implant 5 is shown in front of the iris 3, at the lower temporal side T, spaced apart from the pupil 2. At any rate the or each implant 5 is spaced apart from the pupil, i.e. not in the pupillary axis, in order not to impair sight.

The positions shown in figures 1A-C are merely examples of possible positions of eye implants 5. It will be obvious that also other positions can be possible, whereas other numbers of eye implants 5 can be positioned in the same eye. Also eye implants can be provided in the other eye of the same individual.

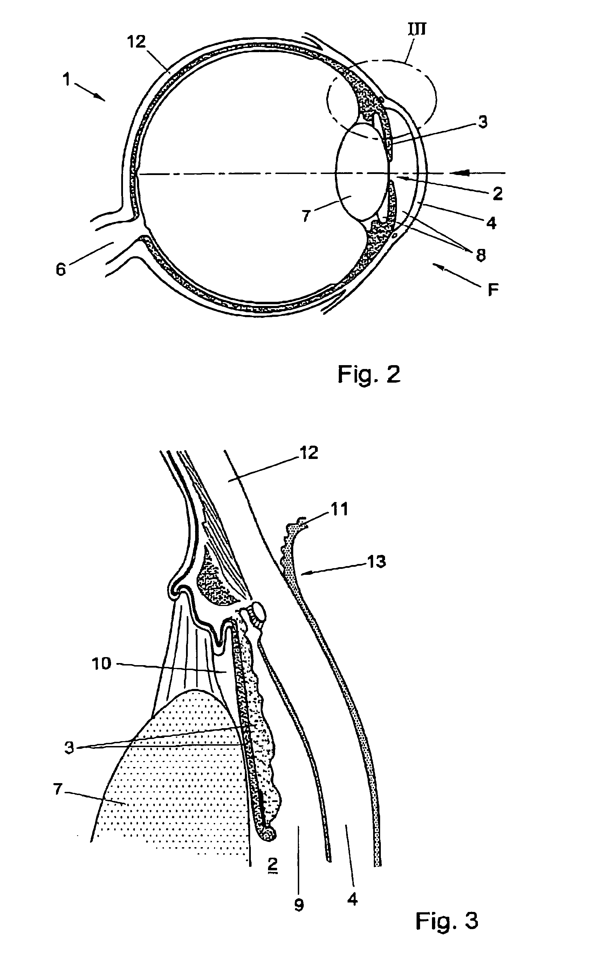

Figure 2 shows a section through a human eye 1, figure 3 shows part of the section shown in figure 2, indicated by the square in figure 2, denominated figure 3. At the frontal side F of the eye 1, opposite the optic nerve 6 the pupil 2 is visible within the iris 3, behind the cornea 4. Between the cornea 4 and the iris 3, as well as between the iris 3 and the lens 7 an aqueous substance 8 is provided within the anterior chamber 9 and posterior chamber 10, respectively called aqueous humor 8. The cornea is clear and is at the limbus 13 continuous with the conjunctiva 11 and the sclera 12. The cornea 4 is more convex than the sclera 12. On each side of the iris 3, that is outside the area defined by the limbus 13, the sclera 12 extends, covered by the conjunctiva 11.

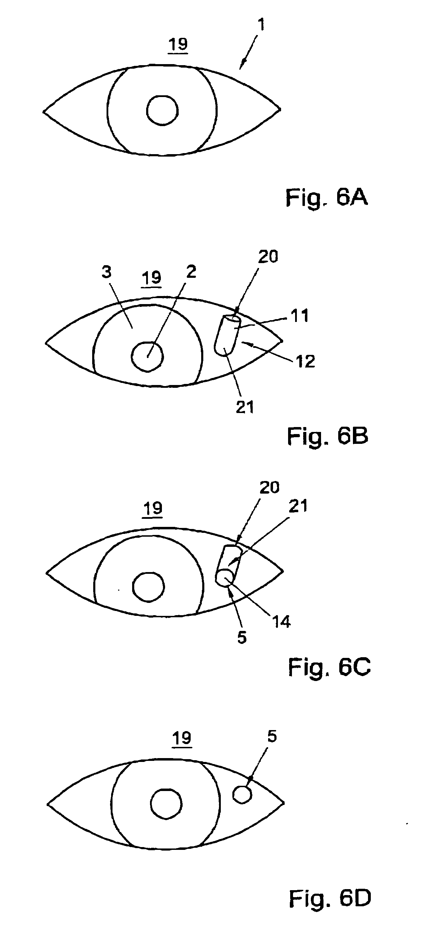

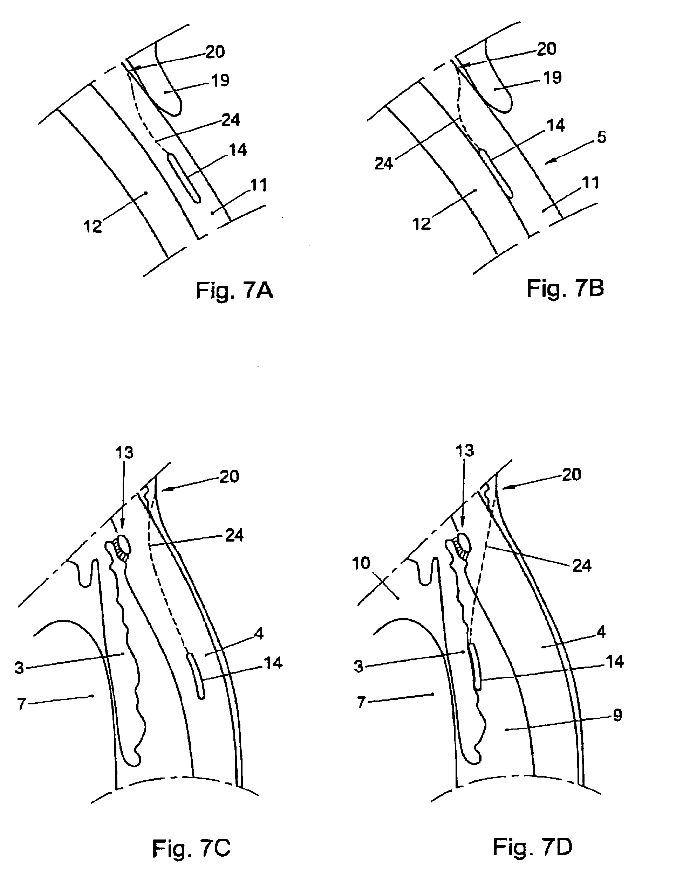

In figure 4 various forms and shapes of eye implants 5 are shown, basically formed by relatively flat elements 14. In figure 4 from left to right the element 14 is shown as a circular element 14, a ring shaped element 14, comprising an opening 15, a circular element 14, comprising a star shaped element 16 printed or otherwise provided thereon or therein and a substantially hart-shaped element 14 with rounded edges 17. These elements 14 are only shown as possible embodiments. Basically any shape and/or design is possible. An element 14 is preferably biocompatible, that is made of a suitable material to be used on or preferably in an eye. The element 14 may be made of a mono material, for example chosen from the group of platinum, platinum-iridium, titanium, gold, silver, glass, polymethylmethacrylate, stone, jewellery stone, sand, silica, porcelain, hair, cilia or can be of two or more materials, preferably chosen from the same group. Also alloys can be used or plastic. An element 14 according to the present invention is preferably made of a substantially non-transparent or opaque material comprising for example light reflecting elements, inserts, prints or the like. Essential of the element 14 is that once positioned on or in an eye 1, as shown in figures 1A - 1C and 7A-7C the eye implant is visible in frontal view and/or light reflecting, that is reflecting light within the spectrum visible for human eyes. In alternative embodiments an element 14 according to the present invention may be made such that it is detectable by for example magnetic or electronic devices.

In figure 5 an element 14 is shown in enlarged cross section along the line V-V in figure 4. The element 14 is flat and comprises a rounded edge 18. The diameter D of the element 14 is less than 5 mm. For an embodiment as shown in figure 1B or 1C the diameter D is preferably less than for an embodiment as shown in figure 1A. The diameter D is preferably within the range of approximately 0.5 and 4 mm, depending on for example the desired position within the eye 1. The thickness H, perpendicular to said diameter D is substantially less than said diameter D. The thickness H is in the embodiment shown for example less than approximately 0.5 mm, preferably between 0 and 0.3 mm. The thickness H may be as small as between 1 and 250 microns. The thickness H has to be such that the element 14 can be inserted into the eye 1, as will be discussed later referring to figures 6 and 7.

The element 14 is preferably made such that it can be sterilized in a suitable manner without damage to said element.

In figure 6A - D four steps are shown schematically for placing an element 14 into an eye 1, for forming an eye implant 5.

In figure 6A the eye 1 is prepared, for example by applying a suitable anaesthetic and antibiotic into the eye. Next the upper eye lid 19 is lifted slightly, and an opening 20 is made in at least the conjunctiva 11 by use of for example a suitable scalpel. In figure 6B the opening 20 is shown, made in the conjunctiva 11 in a position such that this will normally be covered by the eye lid 19. Then the conjunctiva 11 is separated partly from the underlying sclera 12 with a spatula or a (colored) visco elastic material, for example hyaluronic acid in order to form a tunnel shaped pocket 21, ending spaced apart from the pupil 2 and iris 3, for example at the same horizontal level.

Next, as is shown in figure 6C, the element 14 is positioned within the pocket 21, using a suitable forceps, injector or visco elastic or combination thereof, after which the opening 20 is self sealing, enclosing the element 14 for forming the eye implant 5. If at all necessary the opening 20 can be closed off by suitable sutures or any other suitable means.

Finally, the eyelid 19 is returned to the original position and the conjunctiva 11 will return back against the sclera 12, which leaves after a suitable healing period only the eye lid 5 visible in frontal view, as is shown in figure 6D.

In figures 6A - D a method is shown for implanting an element 14, for the formation of an eye implant 5, between the sclera 12 and the conjunctiva 11, as shown in figure 7B, spaced apart from the iris 3, as is shown in figure 1A. However, according to the present invention the eye implant 5 can also be provided by inserting the element 14 in a different manner, for example within the conjunctiva 11, as is shown in figure 7A, within the cornea, in front of the iris 3, as is shown in figure 7C and figure 1B, or on the iris 3, within the interior chamber 9, behind the transparent cornea 4, as is shown in figures 1C and 7D. In figures 7A - 7D the incisions made for placing the element 14 in the respective positions are shown by dotted lines 24, extending between the opening 20 and the element 14.

An element 14 can be made slightly spherical or be provided with a concave recess 25, as schematically shown in figure 5 by the dotted line 26, such that it will connect to the relevant part of the eye 1 more suitably. Also attachment means can be provided (not shown) for connecting said element to eye tissue, for example the iris 13 in a position as shown in figure 7D. Such attachment means can for example be small hooks, claws, glue, pins or the like. The surface 27 of an element 14 which is during use visible may be provided with a print or the like, for example glittering for extra visual effects.

Although in the embodiments shown the implant 5 is provided within the eye 1, that is embedded in eye tissue, it may also be provided at least partly on the outside of the eye, that is on or partly in the conjunctiva and/or the sclera and/or the cornea. In such embodiment it is especially preferred to provide said attachment means in order to prevent migration of said element over the eye, which might impair the normal functions of the eye, for example vision.

One or more elements 14 for forming an eye implant 5 may be sterilized and packed in a container, for example a pouch, sealed against influence from the environment. A suitable instruction may be packed in or with said container, such that the intended use of said element is clear. A method according to the present invention, especially for positioning the element 14 as is shown in figures 7A and 7B and possibly 7C may be performed by any suitable person, for example a tatooist an optician or a surgeon. The eye implant 5 is basically of a cosmetic nature, visible in frontal view and may be reflective for visible light and/or light emitting.

The lower temporal or nasal area provides for a high exposure of the cosmetic implant. The implant is preferably positioned spaced apart from the pupillary axis and placed such that it does not interfere with for example the film of tear-fluid on the cornea.

The visibility of the implant can be provided for or enhanced by for example color, fluorescence, use of different materials, surface finish, prints and combinations thereof.

The implant may also consist of multiple particles, or multiple particles evenly distributed within a carrier substance like a visco elastic, for example hyaluronic acid, that are injected with the carrier into the eye tissue. The particles are non-transparent, for example glittering particles.

The invention is by no means limited to the embodiments as shown and described. Many variations thereof are possible within the scope of the invention.

For example an element according to the present invention may have other forms or dimensions, and may be made of other materials suitable for opthalmic use. When more than one element 14 is positioned in the same eye, these elements may well be interconnected. Openings 20 and incisions or tunnels 21 may be made in other positions and with other means, extending in different directions.

## Claims

1. Eye implant, comprising a flat element made of biocompatible material, said element being made at least partly opaque or light reflecting, such that in use the eye implant shall be visible, once implanted in an eye, **characterized in that** the implant is a cosmetic implant, wherein said element has a relatively small thickness and dimensions perpendicular to said thickness which are less than 5 mm.

2. Eye implant according to claim 1, wherein the implant is designed for altering the normal appearance of an eye.

3. Eye implant according to claim 1 or 2, wherein said element is substantially opaque and made light reflecting.

4. Eye implant according to any one of claims 1-3, wherein said element is substantially disc shaped.

5. Eye implant according to any one of claims 1 - 4, wherein the thickness of said flat element is between 1 and 250 micron.

6. Eye implant according to any one of the previous claims, wherein said dimensions perpendicular to said thickness are between 0.5 and 4 mm.

7. Eye implant according to any one of the previous claims, wherein the implant has an outer edge which is substantially rounded.

8. Eye implant according to any one of the previous claims, wherein the implant is substantially made of a material or combination of materials chosen from the group of platinum, platinum-iridium, titanium, gold, silver, glass, polymethylmethacrylate, stone, jewellery stone, sand, silica, porcelain, hair, cilia.

9. Eye implant according to any one of the previous claims, wherein the material or combination of materials used allows sterilisation.

10. Eye implant according to any one of the previous claims, wherein de implant has at least one concave surface, preferably one concave and one convex surface.

11. Eye implant according to any one of the previous claims, enclosed in a closed container like a box or pouch, said eye implant being sterilised or sterilisable within said container.

12. Eye implant according to any one of the previous claims, wherein attachment means are provided for permanently or releasably connecting said implant to an eye, especially to the conjunctiva, sclera, cornea or iris of an eye.

13. Use of an eye implant according to any one of claims 1- 12, wherein said implant is positioned in an eye, visible in a frontal view of said eye with open eyelids, distanced from the pupil of said eye.

14. Eye implant according to any of claims 1-12, wherein the implant consists of or comprises multiple particles or multiple particles evenly distributed within a carrier substance, for example a visco elastic material, for example hyaluronic acid.

15. Use of a flat, bio compatible element, according to any one of claims 1 - 12 in the preparation of a cosmetic eye implant for implanting in an eye spaced apart from the pupil of the eye.

16. Set of an eye implant according to any one of claims 1 - 12, at least one scalpel or similar knife, visco elastic material and at least one forceps.

## Patentansprüche

1. Augenimplantat mit einem flachen Element aus biokompatiblen Material, wobei das Element mindestens teilweise opak oder lichtreflektierend ausgebildet ist, derart, dass das Augenimplantat bei Benutzung sichtbar ist, nachdem es in das Auge implantiert worden ist,
**dadurch gekennzeichnet, dass** das Implantat ein kosmetisches Implantat ist, wobei das genannte Element eine relativ geringe Dicke und rechtwinklig zu der Dicke verlaufende Bemessungen von weniger als 5 mm hat.

2. Augenimplantat nach Anspruch 1, wobei das Implantat zur Veränderung des normalen Erscheinungsbilds eines Auges ausgebildet ist.

3. Augenimplantat nach Anspruch 1 oder 2, bei dem das Element im Wesentlichen opak und lichtreflektierend ausgebildet ist.

4. Augenimplantat nach einem der Ansprüche 1-3, bei dem das Element im Wesentlichen scheibenförmig ist.

5. Augenimplantat nach einem der Ansprüche 1-4, bei dem die Dicke des flachen Elements zwischen 1 und 250 Mikron beträgt.

6. Augenimplantat nach einem der vorhergehenden Ansprüche, bei dem die rechtwinklig zu der Dicke verlaufenden Bemessungen zwischen 0,5 und 4 mm betragen.

7. Augenimplantat nach einem der vorhergehenden Ansprüche, wobei das Implantat einen Außenrand hat, der im Wesentlichen gerundet ist.

8. Augenimplantat nach einem der vorhergehenden Ansprüche, wobei das Implantat im Wesentlichen aus einem Material oder einer Kombination von Materialien ausgebildet ist, das bzw. die aus der Gruppe gewählt ist bzw. sind, zu der Platin, Platin-Iridium, Titan, Gold, Silber, Glas, Polymethylmethacrylat, Stein, Schmuckstein, Sand, Siliciumdioxid, Porzellan, Haar und Wimpern zählen.

9. Augenimplantat nach einem der vorhergehenden Ansprüche, bei dem das verwendete Material oder die verwendete Kombination von Materialien sterilisierbar ist.

10. Augenimplantat nach einem der vorhergehenden Ansprüche, wobei das Implantat mindestens eine konkave Fläche, vorzugsweise eine konkave und eine konvexe Fläche aufweist.

11. Augenimplantat nach einem der vorhergehenden Ansprüche, untergebracht in einem geschlossenen Behälter wie z.B. einer Box oder einem Beutel, wobei das Augenimplantat in dem Behälter sterilisiert oder sterilisierbar ist.

12. Augenimplantat nach einem der vorhergehenden Ansprüche, bei dem Befestigungsmittel zum dauerhaften oder lösbaren Verbinden des Implantats an einem Auge, insbesondere an der Conjunctiva, Sclera, Kornea oder Iris eines Auges, vorgesehen sind.

13. Verwendung eines Augenimplantats nach einem der Ansprüche 1-12, wobei das Implantat in einem Auge derart positioniert wird, dass es bei frontaler Betrachtung des Auges bei offenen Augenlidern sichtbar ist und von der Pupille des Auges beabstandet ist.

14. Augenimplantat nach einem der Ansprüche 1-12, wobei das Implantat mehrere Partikel oder mehrere gleichmäßig in einer Trägersubstanz verteilte Partikel aufweist, wobei die Trägersubstanz beispielsweise ein viskoelastisches Material wie z.B. Hyaluronsäure ist.

15. Verwendung eines flachen biokompatiblen Elements nach einem der Ansprüche 1-12 bei der Präparation eines kosmetischen Augenimplantats zum Implantieren in einem Auge unter Abstand von der Pupille des Auges.

16. Set aus einem Augenimplantat nach einem der Ansprüche 1-12, mindestens einem Skalpell oder ähnlichem Messer, viskoelastischem Material und mindestens einer Zange.

## Revendications

1. Implant oculaire comprenant un élément plat réalisé avec un matériau biocompatible, ledit élément étant rendu au moins partiellement opaque ou réfléchissant, de sorte qu'à l'usage, l'implant oculaire est visible une fois implanté dans un oeil, **caractérisé en ce que** l'implant est un implant cosmétique, dans lequel ledit élément a une épaisseur relativement petite et des dimensions perpendiculaires à ladite épaisseur qui sont inférieures à 5 mm.

2. Implant oculaire selon la revendication 1, dans lequel l'implant est conçu pour modifier l'apparence normale d'un oeil.

3. Implant oculaire selon la revendication 1 ou 2, dans lequel ledit élément est sensiblement opaque et réfléchissant.

4. Implant oculaire selon l'une quelconque des revendications 1 à 3, dans lequel ledit élément a sensiblement une forme de disque.

5. Implant oculaire selon l'une quelconque des revendications 1 à 4, dans lequel l'épaisseur dudit élément plat est comprise entre 1 et 250 micromètres.

6. Implant oculaire selon l'une quelconque des revendications précédentes, dans lequel lesdites dimensions perpendiculaires à ladite épaisseur sont comprises entre 0,5 et 4 mm.

7. Implant oculaire selon l'une quelconque des revendications précédentes, dans lequel l'implant a un bord externe qui est sensiblement arrondi.

8. Implant oculaire selon l'une quelconque des revendications précédentes, dans lequel l'implant est sensiblement réalisé avec un matériau ou une combinaison de matériaux choisi dans le groupe comprenant le platine, le platine-iridium, le titane, l'or, l'argent, le verre, le polyméthyl méthacrylate, la pierre, les pierres précieuses, le sable, la silice, la porcelaine, les cheveux, les cils.

9. Implant oculaire selon l'une quelconque des revendications précédentes, dans lequel le matériau ou la combinaison de matériaux utilisés permet la stérilisation.

10. Implant oculaire selon l'une quelconque des revendications précédentes, dans lequel l'implant a au moins une surface concave, de préférence une surface concave et une surface convexe.

11. Implant oculaire selon l'une quelconque des revendications précédentes, enfermé dans un récipient hermétique en forme de boîte ou de sachet, ledit implant oculaire étant stérilisé ou stérilisable à l'intérieur dudit récipient.

12. Implant oculaire selon l'une quelconque des revendications précédentes, dans lequel on prévoit des moyens de fixation pour raccorder de manière permanente ou amovible ledit implant à un oeil, en particulier à la conjonctive, la sclère, la cornée ou l'iris d'un oeil.

13. Utilisation d'un implant oculaire selon l'une quelconque des revendications 1 à 12, dans laquelle ledit implant est positionné dans un oeil, visible sur une vue de face dudit oeil avec les paupières ouvertes, à distance de la pupille dudit oeil.

14. Implant oculaire selon l'une quelconque des revendications 1 à 12, dans lequel l'implant se compose ou comprend de multiple particules ou de multiple particules réparties régulièrement avec une substance porteuse, par exemple un matériau viscoélastique, par exemple de l'acide hyaluronique.

15. Utilisation d'un élément plat biocompatible, selon l'une quelconque des revendications 1 à 12 dans la préparation d'un implant oculaire cosmétique pour l'implantation dans un oeil espacé de la pupille de l'oeil.

16. Ensemble d'implant oculaire selon l'une quelconque des revendications 1 à 12, comprenant au moins un scalpel ou une lame similaire, un matériau viscoélastique et au moins une pince.
